(19) Europäisches Patentamt / European Patent Office / Office européen des brevets

(11) **EP 3 249 427 B1**

(12) **EUROPEAN PATENT SPECIFICATION**

(45) Date of publication and mention
of the grant of the patent:
**12.06.2019 Bulletin 2019/24**

(51) Int Cl.:
**G01S 17/74** (2006.01)   **A61B 5/06** (2006.01)
**G01S 17/88** (2006.01)   **G01S 7/486** (2006.01)

(21) Application number: **17167146.4**

(22) Date of filing: **19.04.2017**

(54) **A DEVICE FOR REGENERATING AN INFRARED SIGNAL**

VORRICHTUNG ZU REGENERATION EINES INFRAROTSIGNALS

DISPOSITIF POUR LA REGENERATION D'UN SIGNAL INFRAROUGE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.04.2016 US 201615135658**

(43) Date of publication of application:
**29.11.2017 Bulletin 2017/48**

(73) Proprietor: **Globus Medical, Inc.**
**Audubon, PA 19403 (US)**

(72) Inventors:
• **LE BOEUF, Robert J.**
**Salem, NH 03079 (US)**
• **OLENIO, Zachary**
**Derry, NH 03038 (US)**
• **YAU, James**
**Methuen, MA 01844 (US)**
• **CRAWFORD, Neil R.**
**Chandler, AZ 85224 (US)**

(74) Representative: **Morabito, Sara et al**
**Cantaluppi & Partners S.r.l.**
**Piazzetta Cappellato Pedrocchi, 18**
**35122 Padova (IT)**

(56) References cited:
**US-A1- 2015 192 664**   **US-A1- 2015 260 831**
**US-A1- 2016 235 493**   **US-B1- 8 018 579**

**Description**

FIELD

The present disclosure relates to position recognition systems and in particular, infrared based position recognition system for use with a robot assisted surgery.

BACKGROUND

Position recognition systems are used to determine the position of and track a particular object in 3-dimensions. In robot assisted surgeries, for example, certain objects, such as a surgical instrument, needs to be tracked with a high degree of precision as the instrument is being positioned and moved by a physician.

An infrared signal based position recognition systems use either passive sensors or active sensors. In passive sensors, objects to be tracked such as spherical balls are positioned at strategic locations of the object to be tracked. Infrared transmitters transmit a signal having a predetermined waveform pattern. The spherical balls reflect the signal and the reflected signals are received by infrared signal detectors that are generally located near the transmitters.

In active sensors, the objects to be tracked have their own infrared transmitters and thus generate their own infrared signals in response to the signals transmitted from an infrared transmitter of the position recognition system. US2015260831 A1 discloses optical range finders which are configured to transmit optical bursts toward a target and detect a corresponding received burst, whereby DC offset in the received burst due to square law detection are be offset based on a difference between high pass and low pass filtered portions of the received burst.

In either active or passive sensors, the responsive infrared signals from the objects to be tracked are received and amplified in a form which is as close to the original infrared signals as possible. The amplified signal is then converted into a digital signal which is usable by a signal processor. The system then geometrically resolves the 3-dimensional position of the spherical balls based on one or more of the converted digital signals, cameras, digital waveform shape of the amplified signals, known locations of the spherical balls, distance, the time it took to receive the responsive signals, or a combination thereof.

One problem is that due to attenuation of infrared signals, the conventional position recognition systems have a relatively limited working range of distance between the system and objects to be tracked. Typically, the working range is limited to about 3-5 meters, at best, before the amplified signal loses sufficient original waveform information to be useful.

Moreover, conventional systems require relatively high bandwidth, high slew rate amplifiers which involve complex and expensive circuits. Therefore there is a need to provide an improved system and method for recognizing the 3-dimensional position of an object, which has a longer working range, is accurate, and is less expensive.

SUMMARY

To meet this and other needs, devices, systems, and methods for determining the 3-dimensional position of an object are described. The invention, defined by the claims, relates to an improved device for regenerating an infrared signal transmitted over the air for use in detecting a 3-dimensional position of an object.

According to an embodiment of the invention which is not claimed, a surgical robot system includes a robot having a robot base and a display, a robot arm coupled to the robot base, and an end-effector coupled to the robot arm, the end-effector having one or more tracking markers, wherein movement of the end-effector is electronically controlled by the robot. The system further includes a camera stand including at least one camera able to detect the one or more tracking markers, the camera including at least one infrared signal detector that receives from the one or more tracking markers an infrared signal, wherein the robot determines a 3-dimensional position of the one or more tracking markers based on the infrared signal, and wherein the infrared signal includes a burst signal. The infrared signal may include a burst pattern including the burst signal for a fixed amount of time and a non-burst signal for a fixed amount of time.

According to an embodiment of the invention which is claimed, the regeneration device includes an infrared signal transmitter and an infrared signal detector that receives from the object a responsive infrared signal in response to the infrared signal transmitted by the infrared transmitter. A low pass filter receives the responsive infrared signal from the detector and outputs a low-pass filtered signal. A comparator has a first input connected to the output of the infrared signal detector and a second input connected to the output of the low pass filter. The comparator generates an output representing a logic state based on comparison between the first and second inputs.

Advantageously, the present invention relaxes the requirement of a high bandwidth, high slew output amplification and allows position recognition of the object over a longer distance due to less sensitivity of the signal amplitude variation.

According to a further aspect of the invention it is provided an infrared signal based position recognition system for use with a robot-assisted surgery comprising: an infrared signal transmitter that transmits an infrared signal over the air towards an object whose position is to be determined; an infrared signal receiver that receives from the object a responsive infrared signal in response to the transmitted infrared signal; a low pass filter that receives the responsive infrared signal from the infrared signal receiver and outputs a filtered signal; a comparator that generates a comparator output representing a logic state based on a first input receiving the responsive infrared signal and a second input receiving the filtered signal from the low pass filter; a signal processor adapted to receive the comparator output and determine a 3-dimensional position of the object.

In a version, the system further comprises an amplifier coupled between the infrared signal receiver and the low pass filter.

In a further version the recognition system further comprises: an amplifier coupled between the infrared signal receiver and the low pass filter and operable to generate an amplified responsive infrared signal; and a diode connected between the amplifier and the low pass filter to reduce the voltage of the amplified responsive infrared signal by a predetermined amount.

Advantageously, the system further comprises a diode connected between the amplifier and the low pass filter, wherein the low pass filter includes a capacitor coupled between the diode and ground.

In another version, the low pass filter includes: a voltage divider having first and second resistors connected in series between the output of the infrared signal receiver and ground, a node between the first and second resistors defining an output of the voltage divider; a capacitor coupled between the voltage divider output and ground.

According to a further aspect, which is not claimed, it is provided a surgical robot system comprising: a robot having a robot base and a display, a robot arm coupled to the robot base, and an end-effector coupled to the robot arm, the end-effector having one or more tracking markers, wherein movement of the end-effector is electronically controlled by the robot; and a camera stand including at least one camera able to detect the one or more tracking markers, the camera including at least one infrared signal detector that receives from the one or more tracking markers an infrared signal, wherein the robot determines a 3-dimensional position of the one or more tracking markers based on the infrared signal, and wherein the infrared signal includes a burst signal.

In a version of the system, the infrared signal includes a burst pattern including the burst signal for a fixed amount of time and a non-burst signal for a fixed amount of time.

In a further version the system comprises at least one infrared signal transmitter attached to a portion of the camera stand that transmits an infrared signal towards the one or more tracking markers whose position is to be determined. Advantageously the at least one infrared signal transmitter transmits an infrared signal comprised of a burst signal, a non-burst signal, or a combination of both.

In a version, the one or more tracking markers in the end-effector are active markers having an active state and an inactive state, the active state emitting the infrared signal.

Advantageously, the system further comprises a surgical instrument having one or more tracking markers to be tracked by the robot system, the surgical instrument configured to be positioned in the end-effector in order to align the surgical instrument along a given trajectory for a surgical procedure.

DESCRIPTION OF THE DRAWINGS

FIG. 1 is an overhead view of a potential arrangement for locations of the robotic system, patient, surgeon, and other medical personnel during a surgical procedure;
FIG. 2 illustrates the robotic system including positioning of the surgical robot and the camera relative to the patient according to one embodiment;
FIG. 3 illustrates a surgical robotic system in accordance with an exemplary embodiment;
FIG. 4 illustrates a portion of a surgical robot in accordance with an exemplary embodiment;
FIG. 5 illustrates a block diagram of a surgical robot in accordance with an exemplary embodiment;
FIG. 6 illustrates a surgical robot in accordance with an exemplary embodiment;
FIGS. 7A-7C illustrate an end-effector in accordance with an exemplary embodiment;
FIG. 8 illustrates a surgical instrument and the end effector, before and after, inserting the surgical instrument into the guide tube of the end effector according to one embodiment;
FIGS. 9A-9C illustrate portions of an end-effector and robot arm in accordance with an exemplary embodiment;
FIG. 10 illustrates a dynamic reference array, an imaging array, and other components in accordance with an exemplary embodiment;
FIG. 11 illustrates a method of registration in accordance with an exemplary embodiment;
FIG. 12A-12B illustrate embodiments of imaging devices according to exemplary embodiments;
FIG. 13 is a waveform of an exemplary infrared signal;
FIG. 14 is a functional diagram of an infrared signal regeneration circuit according to the invention;
FIG. 15 is a functional diagram of an infrared signal based position recognition system for use with a robot-assisted surgery according to one aspect; and

FIG. 16 is a graph illustrating signal outputs at various nodes of the infrared signal regeneration circuit of FIG. 14.

DETAILED DESCRIPTION

It is to be understood that the present disclosure is not limited in its application to the details of construction and the arrangement of components set forth in the description herein or illustrated in the drawings. The teachings of the present disclosure may be used and practiced in other embodiments and practiced or carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Unless specified or limited otherwise, the terms "mounted," "connected," "supported," and "coupled" and variations thereof are used broadly and encompass both direct and indirect mountings, connections, supports, and couplings. Further, "connected" and "coupled" are not restricted to physical or mechanical connections or couplings.

The following discussion is presented to enable a person skilled in the art to make and use embodiments of the present disclosure. Various modifications to the illustrated embodiments will be readily apparent to those skilled in the art, and the principles herein can be applied to other embodiments and applications without departing from embodiments of the present disclosure. Thus, the embodiments are not intended to be limited to embodiments shown, but are to be accorded the widest scope consistent with the principles and features disclosed herein. The following detailed description is to be read with reference to the figures, in which like elements in different figures have like reference numerals. The figures, which are not necessarily to scale, depict selected embodiments and are not intended to limit the scope of the embodiments. Skilled artisans will recognize the examples provided herein have many useful alternatives and fall within the scope of the embodiments.

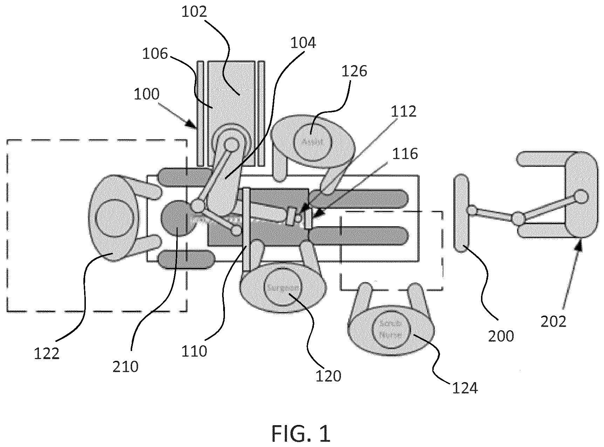

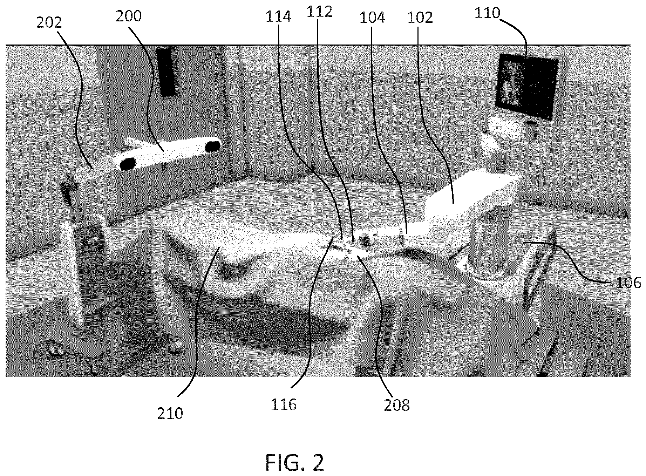

FIGS. 1 and 2 illustrate a surgical robot system 100 in accordance with an exemplary embodiment. Surgical robot system 100 may include, for example, a surgical robot 102, one or more robot arms 104, a base 106, a display 110, an end-effector 112, for example, including a guide tube 114, and one or more tracking markers 118. The surgical robot system 100 may include a patient tracking device 116 also including one or more tracking markers 118, which is adapted to be secured directly to the patient 210 (e.g., to the bone of the patient 210). The surgical robot system 100 may also utilize a camera 200, for example, positioned on a camera stand 202. The camera stand 202 can have any suitable configuration to move, orient, and support the camera 200 in a desired position. The camera 200 may include any suitable camera or cameras, such as one or more infrared cameras (e.g., bifocal cameras), able to identify, for example, active and passive tracking markers 118 in a given measurement volume viewable from the perspective of the camera 200. The camera 200 may scan the given measurement volume and detect the light that comes from the markers 118 in order to identify and determine the position of the markers 118 in three-dimensions. For example, active markers 118 may include infrared-emitting markers that are activated by an electrical signal (e.g., infrared light emitting diodes (LEDs)), and passive markers 118 may include retro-reflective markers that reflect infrared light (e.g., they reflect incoming IR radiation into the direction of the incoming light), for example, emitted by illuminators on the camera 200 or other suitable device.

FIGS. 1 and 2 illustrate a potential configuration for the placement of the surgical robot system 100 in an operating room environment. For example, the robot 102 may be positioned near or next to patient 210. Although depicted near the head of the patient 210, it will be appreciated that the robot 102 can be positioned at any suitable location near the patient 210 depending on the area of the patient 210 undergoing the operation. The camera 200 may be separated from the robot system 100 and positioned at the foot of patient 210. This location allows the camera 200 to have a direct visual line of sight to the surgical field 208. Again, it is contemplated that the camera 200 may be located at any suitable position having line of sight to the surgical field 208. In the configuration shown, the surgeon 120 may be positioned across from the robot 102, but is still able to manipulate the end-effector 112 and the display 110. A surgical assistant 126 may be positioned across from the surgeon 120 again with access to both the end-effector 112 and the display 110. If desired, the locations of the surgeon 120 and the assistant 126 may be reversed. The traditional areas for the anesthesiologist 122 and the nurse or scrub tech 124 remain unimpeded by the locations of the robot 102 and camera 200.

With respect to the other components of the robot 102, the display 110 can be attached to the surgical robot 102 and in other exemplary embodiments, display 110 can be detached from surgical robot 102, either within a surgical room with the surgical robot 102, or in a remote location. End-effector 112 may be coupled to the robot arm 104 and controlled by at least one motor. In exemplary embodiments, end-effector 112 can comprise a guide tube 114, which is able to receive and orient a surgical instrument 608 (described further herein) used to perform surgery on the patient 210. As used herein, the term "end-effector" is used interchangeably with the terms "end-effectuator" and "effectuator element." Although generally shown with a guide tube 114, it will be appreciated that the end-effector 112 may be replaced with any suitable instrumentation suitable for use in surgery. In some embodiments, end-effector 112 can comprise any known structure for effecting the movement of the surgical instrument 608 in a desired manner.

The surgical robot 102 is able to control the translation and orientation of the end-effector 112. The robot 102 is able to move end-effector 112 along x-, y-, and z-axes, for example. The end-effector 112 can be configured for selective rotation about one or more of the x-, y-, and z-axis , and a Z Frame axis (such that one or more of the Euler Angles (e.g., roll, pitch, and/or yaw) associated with end-effector 112 can be selectively controlled). In some exemplary embodiments, selective control of the translation and orientation of end-effector 112 can permit performance of medical procedures with significantly improved accuracy compared to conventional robots that utilize, for example, a six degree of freedom robot arm comprising only rotational axes. For example, the surgical robot system 100 may be used to operate on patient 210, and robot arm 104 can be positioned above the body of patient 210, with end-effector 112 selectively angled relative to the z-axis toward the body of patient 210.

In some exemplary embodiments, the position of the surgical instrument 608 can be dynamically updated so that surgical robot 102 can be aware of the location of the surgical instrument 608 at all times during the procedure. Consequently, in some exemplary embodiments, surgical robot 102 can move the surgical instrument 608 to the desired position quickly without any further assistance from a physician (unless the physician so desires). In some further embodiments, surgical robot 102 can be configured to correct the path of the surgical instrument 608 if the surgical instrument 608 strays from the selected, preplanned trajectory. In some exemplary embodiments, surgical robot 102 can be configured to permit stoppage, modification, and/or manual control of the movement of end-effector 112 and/or the surgical instrument 608. Thus, in use, in exemplary embodiments, a physician or other user can operate the system 100, and has the option to stop, modify, or manually control the autonomous movement of end-effector 112 and/or the surgical instrument 608. Further details of surgical robot system 100 including the control and movement of a surgical instrument 608 by surgical robot 102 can be found in co-pending U.S. patent application Ser. No. 13/924,505, which is incorporated herein by reference in its entirety.

The robotic surgical system 100 can comprise one or more tracking markers 118 configured to track the movement of robot arm 104, end-effector 112, patient 210, and/or the surgical instrument 608 in three dimensions. In exemplary embodiments, a plurality of tracking markers 118 can be mounted (or otherwise secured) thereon to an outer surface of the robot 102, such as, for example and without limitation, on base 106 of robot 102, on robot arm 104, or on the end effector 112. In exemplary embodiments, at least one tracking marker 118 of the plurality of tracking markers 118 can be mounted or otherwise secured to the end-effector 112. One or more tracking markers 118 can further be mounted (or otherwise secured) to the patient 210. In exemplary embodiments, the plurality of tracking markers 118 can be positioned on the patient 210 spaced apart from the surgical field 208 to reduce the likelihood of being obscured by the surgeon, surgical tools, or other parts of the robot 102. Further, one or more tracking markers 118 can be further mounted (or otherwise secured) to the surgical tools 608 (e.g., a screw driver, dilator, implant inserter, or the like). Thus, the tracking markers 118 enable each of the marked objects (e.g., the end effector 112, the patient 210, and the surgical tools 608) to be tracked by the robot 102. In exemplary embodiments, system 100 can use tracking information collected from each of the marked objects to calculate the orientation and location, for example, of the end effector 112, the surgical instrument 608 (e.g., positioned in the tube 114 of the end effector 112), and the relative position of the patient 210.

In exemplary embodiments, one or more of markers 118 may be optical markers. In some embodiments, the positioning of one or more tracking markers 118 on end-effector 112 can maximize the accuracy of the positional measurements by serving to check or verify the position of end-effector 112. Further details of surgical robot system 100 including the control, movement and tracking of surgical robot 102 and of a surgical instrument 608 can be found in co-pending U.S. patent application Ser. No. 13/924,505, which is incorporated herein by reference in its entirety.

Exemplary embodiments include one or more markers 118 coupled to the surgical instrument 608. In exemplary embodiments, these markers 118, for example, coupled to the patient 210 and surgical instruments 608, as well as markers 118 coupled to the end effector 112 of the robot 102 can comprise conventional infrared light-emitting diodes (LEDs) or an Optotrak® diode capable of being tracked using a commercially available infrared optical tracking system such as Optotrak®. Optotrak® is a registered trademark of Northern Digital Inc., Waterloo, Ontario, Canada. In other embodiments, markers 118 can comprise conventional reflective spheres capable of being tracked using a commercially available optical tracking system such as Polaris Spectra. Polaris Spectra is also a registered trademark of Northern Digital, Inc. In an exemplary embodiment, the markers 118 coupled to the end effector 112 are active markers which comprise infrared light-emitting diodes which may be turned on and off, and the markers 118 coupled to the patient 210 and the surgical instruments 608 comprise passive reflective spheres.

In exemplary embodiments, light emitted from and/or reflected by markers 118 can be detected by camera 200 and can be used to monitor the location and movement of the marked objects. In alternative embodiments, markers 118 can comprise a radio-frequency and/or electromagnetic reflector or transceiver and the camera 200 can include or be replaced by a radio-frequency and/or electromagnetic transceiver.

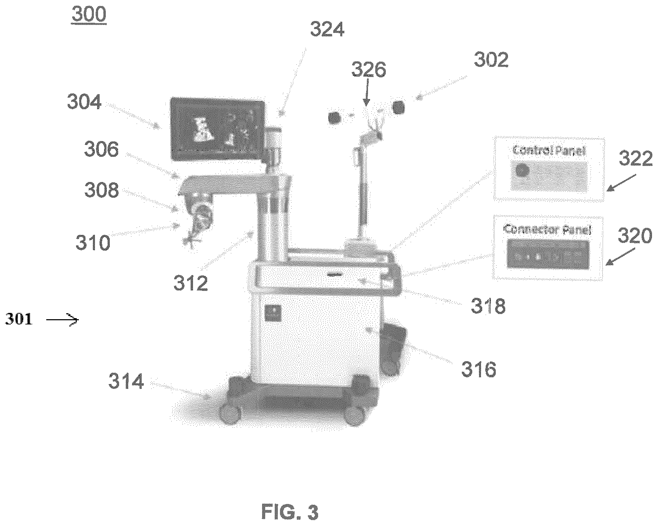

Similar to surgical robot system 100, FIG. 3 illustrates a surgical robot system 300 and camera stand 302, in a docked configuration, consistent with an exemplary embodiment of the present disclosure. Surgical robot system 300 may comprise a robot 301 including a display 304, upper arm 306, lower arm 308, end-effector 310, vertical column 312, casters 314, cabinet 316, tablet drawer 318, connector panel 320, control panel 322, and ring of information 324.

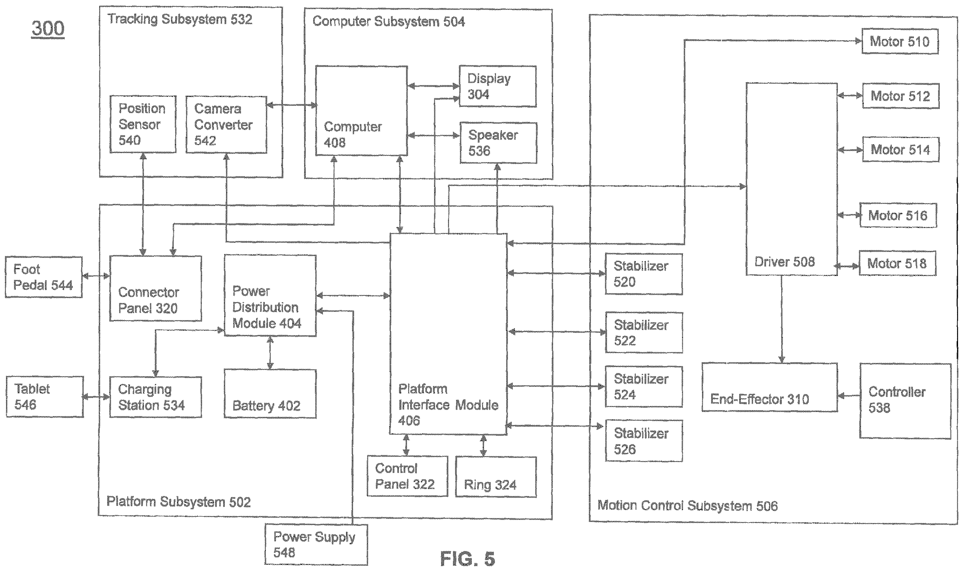

Camera stand 302 may comprise camera 326. These components are described in greater with respect to FIG. 5. FIG. 3 illustrates the surgical robot system 300 in a docked configuration where the camera stand 302 is nested with the robot 301, for example, when not in use. It will be appreciated by those skilled in the art that the camera 326 and robot 301 may be separated from one another and positioned at any appropriate location during the surgical procedure, for example, as shown in FIGS. 1 and 2.

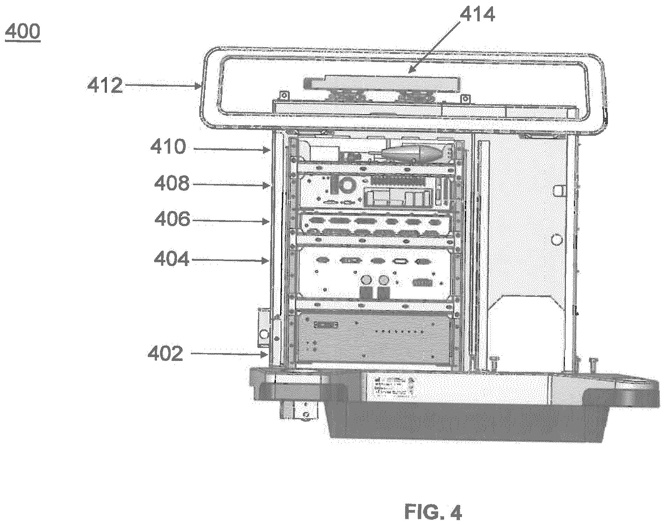

FIG. 4 illustrates a base 400 consistent with an exemplary embodiment of the present disclosure. Base 400 may be a portion of surgical robot system 300 and comprise cabinet 316. Cabinet 316 may house certain components of surgical robot system 300 including but not limited to a battery 402, a power distribution module 404, a platform interface board module 406, a computer 408, a handle 412, and a tablet drawer 414. The connections and relationship between these components is described in greater detail with respect to FIG. 5.

FIG. 5 illustrates a block diagram of certain components of an exemplary embodiment of surgical robot system 300. Surgical robot system 300 may comprise platform subsystem 502, computer subsystem 504, motion control subsystem 506, and tracking subsystem 532. Platform subsystem 502 may further comprise battery 402, power distribution module 404, platform interface board module 406, and tablet charging station 534. Computer subsystem 504 may further comprise computer 408, display 304, and speaker 536. Motion control subsystem 506 may further comprise driver circuit 508, motors 510, 512, 514, 516, 518, stabilizers 520, 522, 524, 526, end-effector 310, and controller 538. Tracking subsystem 532 may further comprise position sensor 540 and camera converter 542. System 300 may also comprise a foot pedal 544 and tablet 546.

Input power is supplied to system 300 via a power source 548 which may be provided to power distribution module 404. Power distribution module 404 receives input power and is configured to generate different power supply voltages that are provided to other modules, components, and subsystems of system 300. Power distribution module 404 may be configured to provide different voltage supplies to platform interface module 406, which may be provided to other components such as computer 408, display 304, speaker 536, driver 508 to, for example, power motors 512, 514, 516, 518 and end-effector 310, motor 510, ring 324, camera converter 542, and other components for system 300 for example, fans for cooling the electrical components within cabinet 316.

Power distribution module 404 may also provide power to other components such as tablet charging station 534 that may be located within tablet drawer 318. Tablet charging station 534 may be in wireless or wired communication with tablet 546 for charging table 546. Tablet 546 may be used by a surgeon consistent with the present disclosure and described herein.

Power distribution module 404 may also be connected to battery 402, which serves as temporary power source in the event that power distribution module 404 does not receive power from input power 548. At other times, power distribution module 404 may serve to charge battery 402 if necessary.

Other components of platform subsystem 502 may also include connector panel 320, control panel 322, and ring 324. Connector panel 320 may serve to connect different devices and components to system 300 and/or associated components and modules. Connector panel 320 may contain one or more ports that receive lines or connections from different components. For example, connector panel 320 may have a ground terminal port that may ground system 300 to other equipment, a port to connect foot pedal 544 to system 300, a port to connect to tracking subsystem 532, which may comprise position sensor 540, camera converter 542, and cameras 326 associated with camera stand 302. Connector panel 320 may also include other ports to allow USB, Ethernet, HDMI communications to other components, such as computer 408.

Control panel 322 may provide various buttons or indicators that control operation of system 300 and/or provide information regarding system 300. For example, control panel 322 may include buttons to power on or off system 300, lift or lower vertical column 312, and lift or lower stabilizers 520-526 that may be designed to engage casters 314 to lock system 300 from physically moving. Other buttons may stop system 300 in the event of an emergency, which may remove all motor power and apply mechanical brakes to stop all motion from occurring. Control panel 322 may also have indicators notifying the user of certain system conditions such as a line power indicator or status of charge for battery 402.

Ring 324 may be a visual indicator to notify the user of system 300 of different modes that system 300 is operating under and certain warnings to the user.

Computer subsystem 504 includes computer 408, display 304, and speaker 536. Computer 504 includes an operating system and software to operate system 300. Computer 504 may receive and process information from other components (for example, tracking subsystem 532, platform subsystem 502, and/or motion control subsystem 506) in order to display information to the user. Further, computer subsystem 504 may also include speaker 536 to provide audio to the user.

Tracking subsystem 532 may include position sensor 504 and converter 542. Tracking subsystem 532 may correspond to camera stand 302 including camera 326 as described with respect to FIG. 3. Position sensor 504 may be camera 326. Tracking subsystem may track the location of certain markers that are located on the different components of system 300 and/or instruments used by a user during a surgical procedure. This tracking may be conducted in a manner consistent with the present disclosure including the use of infrared technology that tracks the location of active or passive elements, such as LEDs or reflective markers, respectively. The location, orientation, and position of structures having these types of markers may be provided to computer 408 which may be shown to a user on display 304. For example, a surgical instrument 608 having these types of markers and tracked in this manner (which may be referred to as a navigational space) may be shown to a user in relation to a three dimensional image of a patient's anatomical structure.

Motion control subsystem 506 may be configured to physically move vertical column 312, upper arm 306, lower arm 308, or rotate end-effector 310. The physical movement may be conducted through the use of one or more motors 510-518. For example, motor 510 may be configured to vertically lift or lower vertical column 312. Motor 512 may be configured to laterally move upper arm 308 around a point of engagement with vertical column 312 as shown in FIG. 3. Motor 514 may be configured to laterally move lower arm 308 around a point of engagement with upper arm 308 as shown in FIG. 3. Motors 516 and 518 may be configured to move end-effector 310 in a manner such that one may control the roll and one may control the tilt, thereby providing multiple angles that end-effector 310 may be moved. These movements may be achieved by controller 538 which may control these movements through load cells disposed on end-effector 310 and activated by a user engaging these load cells to move system 300 in a desired manner.

Moreover, system 300 may provide for automatic movement of vertical column 312, upper arm 306, and lower arm 308 through a user indicating on display 304 (which may be a touchscreen input device) the location of a surgical instrument or component on three dimensional image of the patient's anatomy on display 304. The user may initiate this automatic movement by stepping on foot pedal 544 or some other input means.

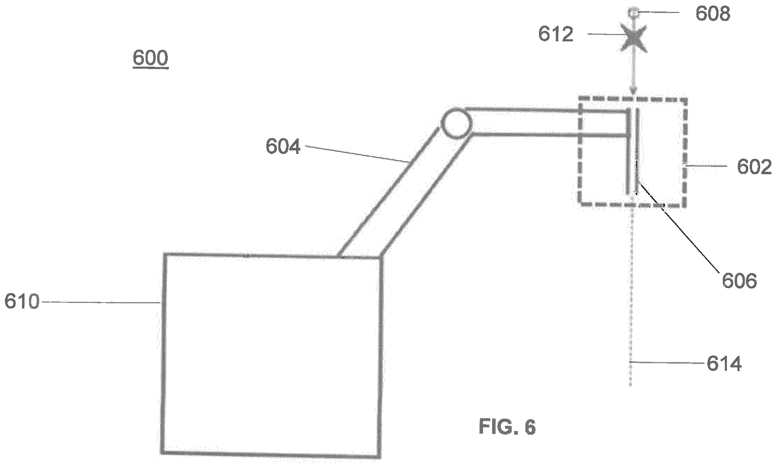

FIG. 6 illustrates a surgical robot system 600 consistent with an exemplary embodiment. Surgical robot system 600 may comprise end-effector 602, robot arm 604, guide tube 606, instrument 608, and robot base 610. Instrument tool 608 may be attached to a tracking array 612 including one or more tracking markers (such as markers 118) and have an associated trajectory 614. Trajectory 614 may represent a path of movement that instrument tool 608 is configured to travel once it is positioned through or secured in guide tube 606, for example, a path of insertion of instrument tool 608 into a patient. In an exemplary operation, robot base 610 may be configured to be in electronic communication with robot arm 604 and end-effector 602 so that surgical robot system 600 may assist a user (for example, a surgeon) in operating on the patient 210. Surgical robot system 600 may be consistent with previously described surgical robot system 100 and 300.

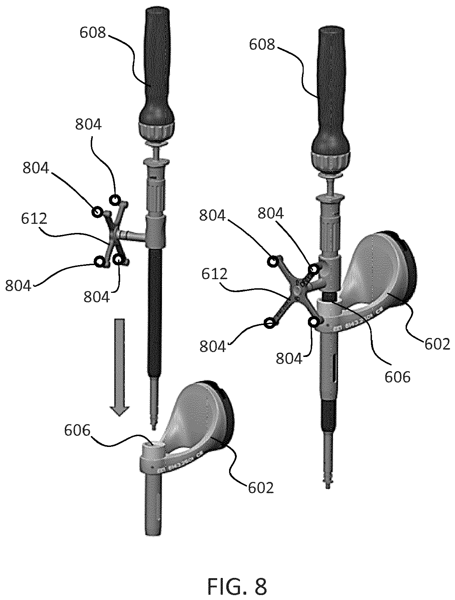

A tracking array 612 may be mounted on instrument 608 to monitor the location and orientation of instrument tool 608. The tracking array 612 may be attached to an instrument 608 and may comprise tracking markers 804. As best seen in FIG. 8, tracking markers 804 may be, for example, light emitting diodes and/or other types of reflective markers (e.g., markers 118 as described elsewhere herein). The tracking devices may be one or more line of sight devices associated with the surgical robot system. As an example, the tracking devices may be one or more cameras 200, 326 associated with the surgical robot system 100, 300 and may also track tracking array 612 for a defined domain or relative orientations of the instrument 608 in relation to the robot arm 604, the robot base 610, end-effector 602, and/or the patient 210. The tracking devices may be consistent with those structures described in connection with camera stand 302 and tracking subsystem 532.

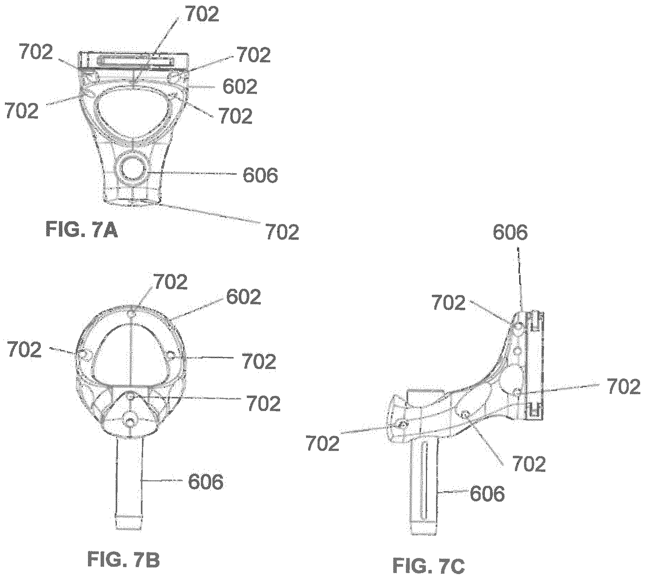

FIGS. 7A, 7B, and 7C illustrate a top view, front view, and side view, respectively, of end-effector 602 consistent with an exemplary embodiment. End-effector 602 may comprise one or more tracking markers 702. Tracking markers 702 may be light emitting diodes or other types of active and passive markers, such as tracking markers 118 that have been previously described. In an exemplary embodiment, the tracking markers 702 are active infrared-emitting markers that are activated by an electrical signal (e.g., infrared light emitting diodes (LEDs)). Thus, tracking markers 702 may be activated such that the infrared markers 702 are visible to the camera 200, 326 or may be deactivated such that the infrared markers 702 are not visible to the camera 200, 326. Thus, when the markers 702 are active, the end effector 602 may be controlled by the system 100, 300, 600, and when the markers 702 are deactivated, the end effector 602 may be locked in position and unable to be moved by the system 100, 300, 600.

Markers 702 may be disposed on or within end-effector 602 in a manner such that the markers 702 are visible by one or more cameras 200, 326 or other tracking devices associated with the surgical robot system 100, 300, 600. The camera 200, 326 or other tracking devices may track end-effector 602 as it moves to different positions and viewing angles by following the movement of tracking markers 702. The location of markers 702 and/or end-effector 602 may be shown on a display 110, 304 associated with the surgical robot system 100, 300, 600, for example, display 110 as shown in FIG. 2 and/or display 304 shown in FIG. 3. This display 110, 304 may allow a user to ensure that end-effector 602 is in a desirable position in relation to robot arm 604, robot base 610, the patient 210, and/or the user.

For example, as shown in FIG. 7A, markers 702 may be placed around the surface of end-effector 602 so that a tracking device placed away from the surgical field 208 and facing toward the robot 102, 301 and the camera 200, 326 is able to view at least 3 of the markers 702 through a range of common orientations of the end effector 602 relative to the tracking device 100, 300, 600. For example, distribution of markers 702 in this way allows end-effector 602 to be monitored by the tracking devices when end-effector 602 is translated and rotated in the surgical field 208.

In addition, in exemplary embodiments, end-effector 602 may be equipped with infrared (IR) receivers that can detect when an external camera 200, 326 is getting ready to read markers 702. Upon this detection, end-effector 602 may then illuminate markers 702. The detection by the IR receivers that the external camera200, 326 is ready to read markers 702 may signal the need to synchronize a duty cycle of markers 702, which may be light emitting diodes, to an external camera200, 326. This may also allow for lower power consumption by the robotic system as a whole, whereby markers 702 would only be illuminated at the appropriate time instead of being illuminated continuously. Further, in exemplary embodiments, markers 702 may be powered off to prevent interference with other navigation tools, such as different types of surgical instruments 608.

FIG. 8 depicts one type of surgical instrument 608 including a tracking array 612 and tracking markers 804. Tracking markers 804 may be of any type described herein including but not limited to light emitting diodes or reflective spheres. Markers 804 are monitored by tracking devices associated with the surgical robot system 100, 300, 600 and may be one or more of the line of sight cameras 200, 326. The cameras 200, 326 may track the location of instrument 608 based on the position and orientation of tracking array 612 and markers 804. A user, such as a surgeon 120, may orient instrument 608 in a manner so that tracking array 612 and markers 804 are sufficiently recognized by the tracking device or camera 200, 326 to display instrument 608 and markers 804 on, for example, display 110 of the exemplary surgical robot system.

The manner in which a surgeon 120 may place instrument 608 into guide tube 606 of the end effector 602 and adjust the instrument 608 is evident in FIG. 8. The hollow tube or guide tube 114, 606 of the end-effector 112, 310, 602 is sized and configured to receive at least a portion of the surgical instrument 608. The guide tube 114, 606 is configured to be oriented by the robot arm 104 such that insertion and trajectory for the surgical instrument 608 is able to reach a desired anatomical target within or upon the body of the patient 210. The surgical instrument 608 may include at least a portion of a generally cylindrical instrument. Although a screw driver is exemplified as the surgical tool 608, it will be appreciated that any suitable surgical tool 608 may be positioned by the end-effector 602. By way of example, the surgical instrument 608 may include one or more of a guide wire, cannula, a retractor, a drill, a reamer, a screw driver, an insertion tool, a removal tool, or the like. Although the hollow tube 114, 606 is generally shown as having a cylindrical configuration, it will be appreciated by those of skill in the art that the guide tube 114, 606 may have any suitable shape, size and configuration desired to accommodate the surgical instrument 608 and access the surgical site.

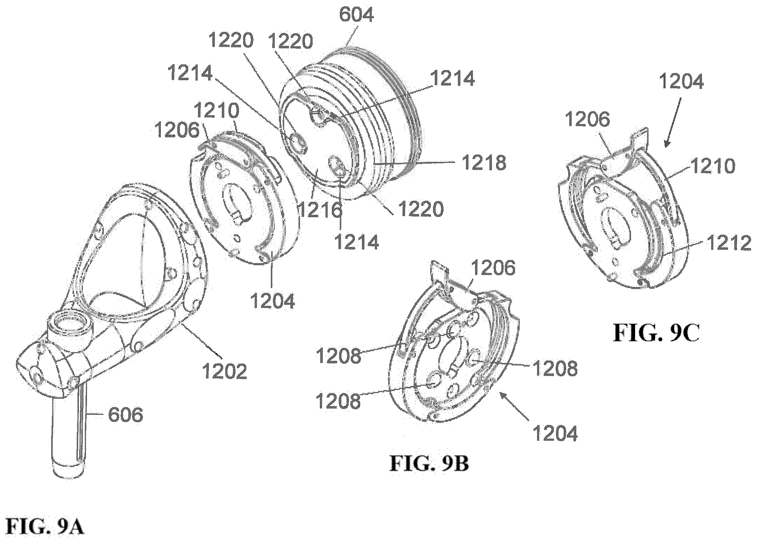

FIGS. 9A-9C illustrate end-effector 602 and a portion of robot arm 604 consistent with an exemplary embodiment. End-effector 602 may further comprise body 1202 and clamp 1204. Clamp 1204 may comprise handle 1206, balls 1208, spring 1210, and lip 1212. Robot arm 604 may further comprise depressions 1214, mounting plate 1216, lip 1218, and magnets 1220.

End-effector 602 may mechanically interface and/or engage with the surgical robot system and robot arm 604 through one or more couplings. For example, end-effector 602 may engage with robot arm 604 through a locating coupling and/or a reinforcing coupling. Through these couplings, end-effector 602 may fasten with robot arm 604 outside a flexible and sterile barrier. In an exemplary embodiment, the locating coupling may be a magnetically kinematic mount and the reinforcing coupling may be a five bar over center clamping linkage.

With respect to the locating coupling, robot arm 604 may comprise mounting plate 1216, which may be non-magnetic material, one or more depressions 1214, lip 1218, and magnets 1220. Magnet 1220 is mounted below each of depressions 1214. Portions of clamp 1204 may comprise magnetic material and be attracted by one or more magnets 1220. Through the magnetic attraction of clamp 1204 and robot arm 604, balls 1208 become seated into respective depressions 1214. For example, balls 1208 as shown in FIG. 9B would be seated in depressions 1214 as shown in FIG. 9A. This seating may be considered a magnetically-assisted kinematic coupling. Magnets 1220 may be configured to be strong enough to support the entire weight of end-effector 602 regardless of the orientation of end-effector 602. The locating coupling may be any style of kinematic mount that uniquely restrains six degrees of freedom.

With respect to the reinforcing coupling, portions of clamp 1204 may be configured to be a fixed ground link and as such clamp 1204 may serve as a five bar linkage. Closing clamp handle 1206 may fasten end-effector 602 to robot arm 604 as lip 1212 and lip 1218 engage clamp 1204 in a manner to secure end-effector 602 and robot arm 604. When clamp handle 1206 is closed, spring 1210 may be stretched or stressed while clamp 1204 is in a locked position. The locked position may be a position that provides for linkage past center. Because of a closed position that is past center, the linkage will not open absent a force applied to clamp handle 1206 to release clamp 1204. Thus, in a locked position end-effector 602 may be robustly secured to robot arm 604.

Spring 1210 may be a curved beam in tension. Spring 1210 may be comprised of a material that exhibits high stiffness and high yield strain such as virgin PEEK (poly-ether-ether-ketone). The linkage between end-effector 602 and robot arm 604 may provide for a sterile barrier between end-effector 602 and robot arm 604 without impeding fastening of the two couplings.

The reinforcing coupling may be a linkage with multiple spring members. The reinforcing coupling may latch with a cam or friction based mechanism. The reinforcing coupling may also be a sufficiently powerful electromagnet that will support fastening end-effector 102 to robot arm 604. The reinforcing coupling may be a multi-piece collar completely separate from either end-effector 602 and/or robot arm 604 that slips over an interface between end-effector 602 and robot arm 604 and tightens with a screw mechanism, an over center linkage, or a cam mechanism.

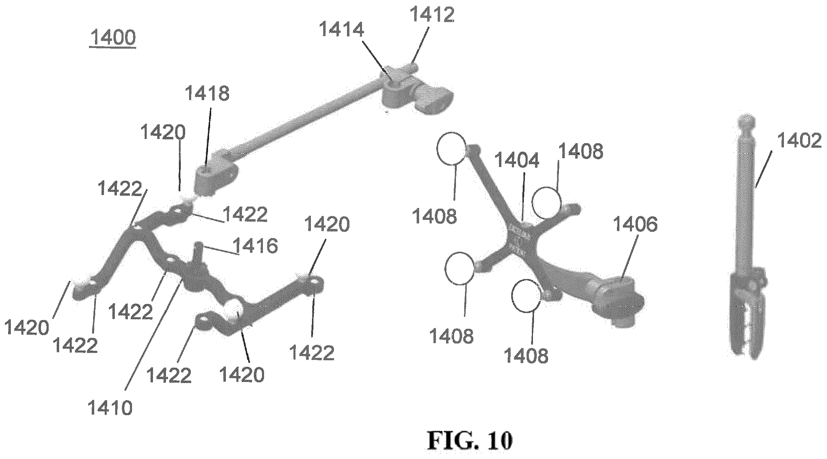

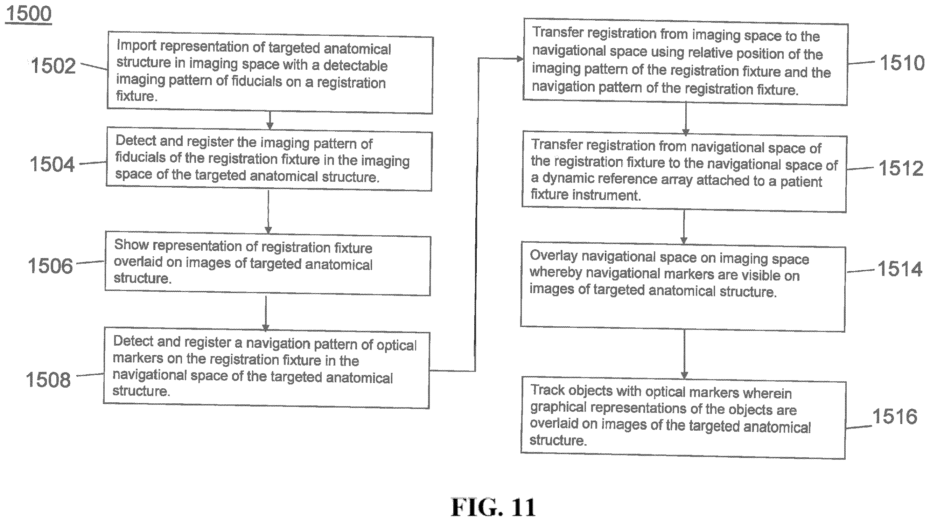

Referring to FIGS. 10 and 11, prior to or during a surgical procedure, certain registration procedures may be conducted in order to track objects and a target anatomical structure of the patient 210 both in a navigation space and an image space. In order to conduct such registration, a registration system 1400 may be used as illustrated in FIG. 10.

In order to track the position of the patient 210, a patient tracking device 116 may include a patient fixation instrument 1402 to be secured to a rigid anatomical structure of the patient 210 and a dynamic reference base (DRB) 1404 may be securely attached to the patient fixation instrument 1402. For example, patient fixation instrument 1402 may be inserted into opening 1406 of dynamic reference base 1404. Dynamic reference base 1404 may contain markers 1408 that are visible to tracking devices, such as tracking subsystem 532. These markers 1408 may be optical markers or reflective spheres, such as tracking markers 118, as previously discussed herein.

Patient fixation instrument 1402 is attached to a rigid anatomy of the patient 210 and may remain attached throughout the surgical procedure. In an exemplary embodiment, patient fixation instrument 1402 is attached to a rigid area of the patient 210, for example a bone that is located away from the targeted anatomical structure subject to the surgical procedure. In order to track the targeted anatomical structure, dynamic reference base 1404 is associated with the targeted anatomical structure through the use of a registration fixture that is temporarily placed on or near the targeted anatomical structure in order to register the dynamic reference base 1404 with the location of the targeted anatomical structure.

A registration fixture 1410 is attached to patient fixation instrument 1402 through the use of a pivot arm 1412. Pivot arm 1412 is attached to patient fixation instrument 1402 by inserting patient fixation instrument 1402 through an opening 1414 of registration fixture 1410. Pivot arm 1412 is attached to registration fixture 1410 by, for example, inserting a knob 1416 through an opening 1418 of pivot arm 1412.

Using pivot arm 1412, registration fixture 1410 may be placed over the targeted anatomical structure and its location may be determined in an image space and navigation space using tracking markers 1420 and/or fiducials 1422 on registration fixture 1410. Registration fixture 1410 may contain a collection of markers 1420 that are visible in a navigational space (for example, markers 1420 may be detectable by tracking subsystem 532). Tracking markers 1420 may be optical markers visible in infrared light as previously described herein. Registration fixture 1410 may also contain a collection of fiducials 1422, for example, such as bearing balls, that are visible in an imaging space (for example, a three dimension CT image). As described in greater detail with respect to FIG. 11, using registration fixture 1410, the targeted anatomical structure may be associated with dynamic reference base 1404 thereby allowing depictions of objects in the navigational space to be overlaid on images of the anatomical structure. Dynamic reference base 1404, located at a position away from the targeted anatomical structure, may become a reference point thereby allowing removal of registration fixture 1410 and/or pivot arm 1412 from the surgical area.

FIG. 11 provides an exemplary method 1500 for registration consistent with the present disclosure. Method 1500 begins at step 1502 wherein a graphical representation (or image(s)) of the targeted anatomical structure may be imported into system 100, 300 600, for example computer 408. The graphical representation may be three dimensional CT or a fluoroscope scan of the targeted anatomical structure of the patient 210 which includes registration fixture 1410 and a detectable imaging pattern of fiducials 1420.

At step 1504, an imaging pattern of fiducials 1420 is detected and registered in the imaging space and stored in computer 408. Optionally, at this time at step 1506, a graphical representation of the registration fixture 1410 may be overlaid on the images of the targeted anatomical structure.

At step 1508, a navigational pattern of registration fixture 1410 is detected and registered by recognizing markers 1420. Markers 1420 may be optical markers that are recognized in the navigation space through infrared light by tracking subsystem 532 via position sensor 540. Thus, the location, orientation, and other information of the targeted anatomical structure is registered in the navigation space. Therefore, registration fixture 1410 may be recognized in both the image space through the use of fiducials 1422 and the navigation space through the use of markers 1420. At step 1510, the registration of registration fixture 1410 in the image space is transferred to the navigation space. This transferal is done, for example, by using the relative position of the imaging pattern of fiducials 1422 compared to the position of the navigation pattern of markers 1420.

At step 1512, registration of the navigation space of registration fixture 1410 (having been registered with the image space) is further transferred to the navigation space of dynamic registration array 1404 attached to patient fixture instrument 1402. Thus, registration fixture 1410 may be removed and dynamic reference base 1404 may be used to track the targeted anatomical structure in both the navigation and image space because the navigation space is associated with the image space.

At steps 1514 and 1516, the navigation space may be overlaid on the image space and objects with markers visible in the navigation space (for example, surgical instruments 608 with optical markers 804). The objects may be tracked through graphical representations of the surgical instrument 608 on the images of the targeted anatomical structure.

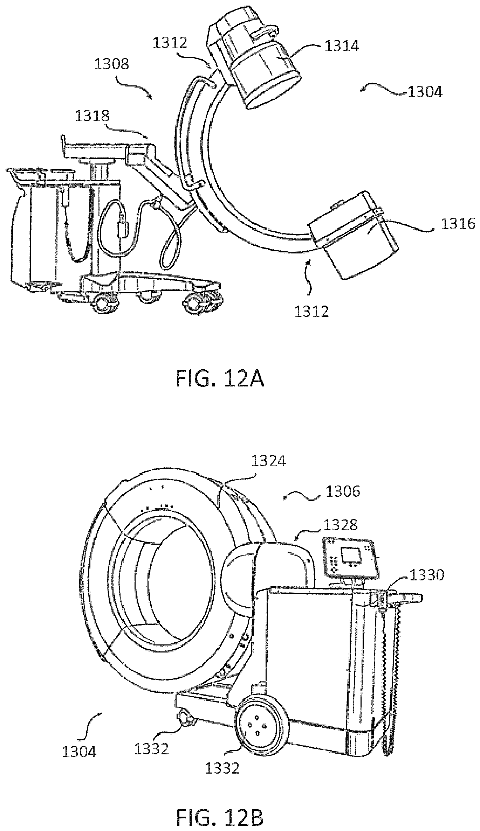

FIGS. 12A-12B illustrate imaging devices 1304 that may be used in conjunction with robot systems 100, 300, 600 to acquire pre-operative, intraoperative, post-operative, and/or real-time image data of patient 210. Any appropriate subject matter may be imaged for any appropriate procedure using the imaging system 1304. The imaging system 1304 may be any imaging device such as imaging device 1306 and/or a C-arm 1308 device. It may be desirable to take x-rays of patient 210 from a number of different positions, without the need for frequent manual repositioning of patient 210 which may be required in an x-ray system. As illustrated in Figure 12A, the imaging system 1304 may be in the form of a C-arm 1308 that includes an elongated C-shaped member terminating in opposing distal ends 1312 of the "C" shape. C-shaped member 1130 may further comprise an x-ray source 1314 and an image receptor 1316. The space within C-arm 1308 of the arm may provide room for the physician to attend to the patient substantially free of interference from x-ray support structure 1318. As illustrated in FIG. 12B, the imaging system may include imaging device 1306 having a gantry housing 1324 attached to a support structure imaging device support structure 1328, such as a wheeled mobile cart 1330 with wheels 1332, which may enclose an image capturing portion, not illustrated. The image capturing portion may include an x-ray source and/or emission portion and an x-ray receiving and/or image receiving portion, which may be disposed about one hundred and eighty degrees from each other and mounted on a rotor (not illustrated) relative to a track of the image capturing portion. The image capturing portion may be operable to rotate three hundred and sixty degrees during image acquisition. The image capturing portion may rotate around a central point and/or axis, allowing image data of patient 210 to be acquired from multiple directions or in multiple planes. Although certain imaging systems 1304 are exemplified herein, it will be appreciated that any suitable imaging system may be selected by one of ordinary skill in the art.

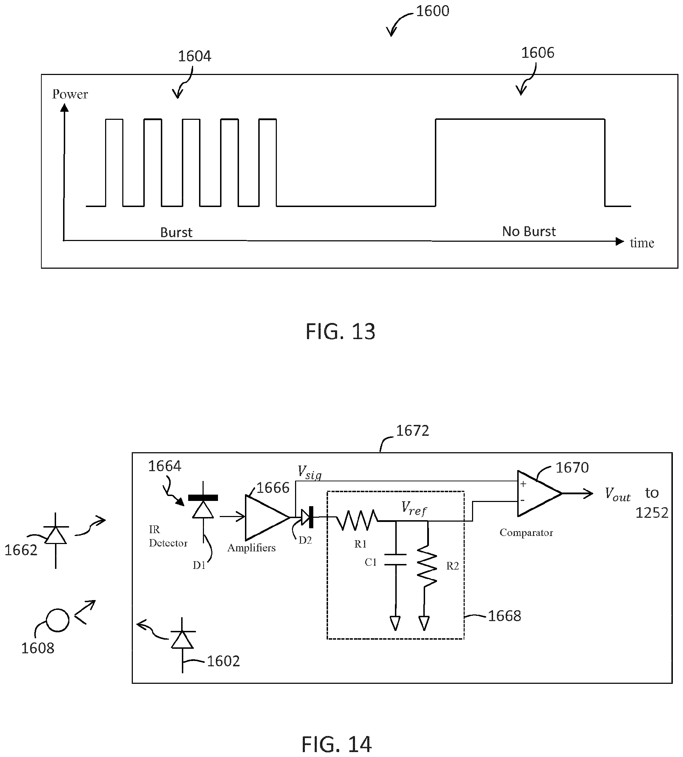

Turning now to FIGS. 13-16, the surgical robot system 100, 300, 600 relies on accurate positioning of the end-effector 112, 602, surgical instruments 608, and the patient 210 (e.g., patient tracking device 116) relative to the desired surgical area. As best seen in FIG. 14, an infrared signal based method may be used to determine the 3-dimensional position of the tracking markers described herein, such as markers 118, 702 and the like. In an exemplary embodiment, the infrared signal method described herein may be especially suitable with active markers 702 on the end-effector 112, 602 and/or passive markers 118 on the surgical instrument 608 or the patient tracking device 116.

As shown in FIGS. 13 and 14, an infrared signal transmitter (e.g., infrared LED) 1602 is capable of transmitting an infrared signal 1600 which can be comprised of a burst signal 1604, non-burst signal 1606, or a combination of both as shown. The infrared signal 1600 may be in the form of a burst pattern. For example, the burst pattern may include the burst signal 1604 for a fixed amount of time and a non-burst signal 1606 for a fixed amount of time. This burst pattern may repeat for a given amount of time. The burst pattern may include any suitable combination of the burst signal 1604 and the non-burst signal 1606 for any suitable duration. For example, the frequency of the burst signal 1604 can be about 110 KHz with a burst duration of about 340 $\mu$sec. The duration of the non-burst signal 1606 can be about 730 $\mu$sec. Infrared signal transmitters 1602 and infrared signal detectors D1 can be incorporated with the camera or cameras 200, 326. Alternatively, the infrared signal transmitters 1602 and infrared signal detectors D1 can be attached to a portion of the camera stand 202, for example, near or proximate to the camera or cameras 200, 326.

The robot-assisted surgery system 100, 300, 600 uses an improved circuit/device 1672 for regenerating an infrared signal transmitted over the air for use in determining a 3-dimensional position of an object, such as the surgical tool 608. The infrared signal regeneration device 1672 of FIG. 14 amplifies the received infrared signal as Vsig and creates a reference signal Vref which is slightly below Vsig. The regeneration device 1672 then peak detects the amplitude of Vsig by comparing its level to Vref, thus creating a toggling voltage for the burst signal 1604, and a single edge for the non-burst signal 1606.

As previously described, tracking markers 118, 702, such as spherical balls (objects to be tracked) 208 can be positioned on the surgical tool 608, the end-effector 112, 602, the patient 210, or other suitable locations. As identified in FIG. 14 as spherical ball 1608, the transmitted signal 1600 may be reflected from the one or more spherical balls 1608 and the reflected signal in the form of a responsive signal 1664 is received by infrared detectors D1. Alternatively, in a system with active sensors, for example, tracking markers 702 on end-effector 601 in FIGS. 7A-7C, the objects 1608 include their own infrared transmitters 1662 which transmit an infrared signal with a known pattern (e.g., the same waveform pattern as received) in response to the infrared signal transmitted by the transmitters 1602.

A responsive infrared signal 1664 (transmitted by infrared transmitters in the objects 1608 in response to the signal from the infrared transmitter 1602 in the case of an active sensor or reflected by the objects from the signal from the infrared transmitter 1602 in the case of a passive sensor) is received by the infrared detectors D1 in analog form. While only one is shown in FIG. 14 for illustrative purpose, there are typically multiple infrared detectors that are strategically positioned in the robotic system 100, 300, 600.

The analog signal 1664 received by the infrared detectors D1 is then fed into an amplifier 1666. The amplifier can be, for example, a relatively inexpensive operational amplifier, such as part no. OPA2381 from Texas Instruments of Dallas, Texas. Although only one amplifier 1666 is shown, amplification of the analog signal 1664 may involve multiple amplifiers that are coupled in parallel or in series or both.

The output of the amplifier 1666 is fed into a diode D2 which is connected to a low pass filter 1668. The diode provides a small voltage drop to prepare the signal for comparison against the original output Vsig of the amplifier 1666. The diode D2 can be, for example, a Schottky diode having a forward bias voltage of about 400 mV. A Schottky diode may be preferable to a normal p-n junction type diode due to its fast response time.

The low pass filter 1668 includes an RC circuit (resistor R1, capacitor C1) and resistor R2 connected in parallel to the capacitor C1. The resistors R1 and R2 act as a voltage divider to divide the output of the diode D2. The values of the resistors R1, R2 can be, for example, 300 Ohms and 5 KOhms, respectively. Accordingly, most of the voltage drop occurs over the resistor R2.

As can be seen in FIG. 14, the output Vref of the low pass filter 1668 is the same as the voltage divider output. The output Vref is fed into one input (-input) of a comparator 1670 while the output of the amplifier 16266 is fed into the other input (+ input) of the comparator. The comparator 1670 can be, for example, a CMOS comparator, such as part no. TLV7211 from Texas Instruments of Dallas, Texas.

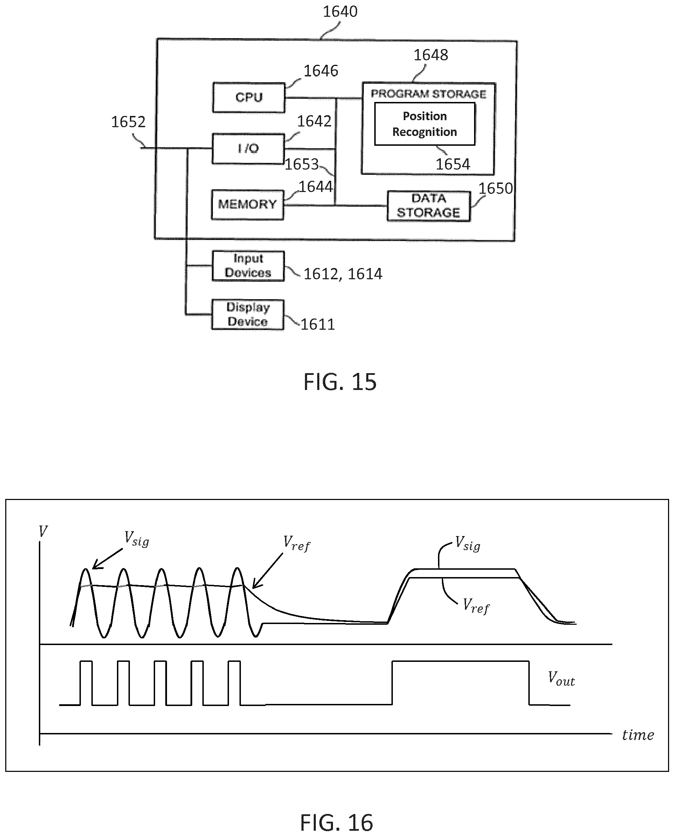

Referring to FIGS. 14 and 16, the operation of the signal regeneration circuit 1672 will now be described. At the output of the amplifier 1666, the amplified signal may not be a perfectly scaled replica of the input due to bandwidth and output slew limitations of the amplifier. According to one aspect of the present invention, however, this is acceptable for reasons now described.

D2, R1, C1 and R2 form a passive peak detector, and a voltage level $\overline{V}_{ref}$ is generated. Its average value is:

$$\overline{V}_{ref} = V_{sigpk} \cdot \frac{R2}{R2+R1} - V_{D2f}$$

(1)

Where:

$V_{sigpk}$ = peak voltage of the amplified infrared signal Vsig;
$V_{D2f}$ = forward voltage of D2.

The voltage signal Vref is fed to the comparator 1670 and is compared against Vsig. Based on the comparison, the comparator 1670 generates a voltage Vout representing the low and high logic states which can be used by a digital signal processor for determining the 3-dimensional positions of the various objects being tracked. In other words, the comparator 1670 converts an analog signal into a digital signal, which can be easily processed by a processor 1646 such as a microcontroller or similar devices.

FIG. 16 shows the waveforms Vsig, Vref and Vout when the infrared detector D1 receives the responsive infrared signal 1664 which may be a reflection of the infrared signal 1600 transmitted by the infrared transmitter 1602. Due to limitations of the bandwidth and output slew of the amplifier, the signal Vsig appears as a sinusoidal wave, rather than the original signal 1600 with sharply defined leading and falling edges.

The signal Vref is lower in voltage than the signal Vsig. The amount of the voltage drop is predetermined based on the forward bias voltage drop of the diode D2 and the voltage drop across the resistor R1. The signal Vref has a relatively flat waveform due to the low pass filter 268.

The comparator 1670 generates a logic high signal (e.g., 5 Volt signal) when Vsig is greater than Vsig and a logic low signal (e.g., 0 Volt signal) when Vsig falls below Vref. As can be seen, the voltage at Vref is proportional to Vsig. At lower voltages (i.e., when the distance between the object 1608 and the infrared detector D1 is relatively long, the difference between Vsig and Vref may not be sufficient to toggle the comparator 1670 without the presence of the diode D2. The diode D2 ensures that there is at least a preset voltage difference between Vsig and Vref (i.e., forward bias voltage of diode D2). Thus, the presence of a diode D2 between the operational amplifier 1666 and the low pass filter 1668 may be important.

As can be seen by the waveform Vout in FIG. 16, it generates a fairly accurate regeneration of the original signal 1600 over longer distance between the infrared transmitter 1602 and the objects 1608 even though the responsive signal 1664 has been substantially degraded.

Advantageously, this configuration relaxes the requirement of a high bandwidth, high slew output amplification. It is also not as sensitive to amplitude variations as simple amplification would be. This is because as Vsig decreases when the distance between the objects 1608 and infrared transmitter 1602 becomes longer, Vref also decreases. That allows the comparator 1670 to continue to toggle correctly when needed. This results in the effective working distance between the objects 1608 and infrared transmitter 1602 to increase by 75%-200% (7-10 meters).

FIG. 15 is a functional diagram of an infrared signal based position recognition system 1640 for use with a robot-assisted surgery according to an aspect of the present invention.

The system 1640 is connected to the output of the comparator 1670 through a communication link 1652 which is connected to an I/O interface 1642, which receives information from and sends information over the communication link 1652. The system 1640 includes memory storage 1644 such as RAM (random access memory), processor (CPU) 1646, program storage 1648 such as FPGA, ROM or EEPROM, and data storage 1650 such as a hard disk, all commonly connected to each other through a bus 1653. The program storage 1648 stores, among others, a position recognition module 1654 containing software to be executed by the processor 1646. The position recognition module 1654 receives the digital waveform Vout through the communication link 1652 and determines the 3-dimensional position of the object 1608 based on the digital waveform, shape of the amplified signals, known locations of the objects and infrared detectors D1, and possibly the time it took to receive the responsive signals 1664. The position recognition module 1654 may also determine the 3-dimensional position of the objects 1608 with the help of the cameras 200, 326 (e.g. stereo cameras) and the tracking system 100, 300, 600. The camera or cameras 200, 326 can record regular optical images or infrared images or both.

The position recognition module 1654 also determines whether the objects being tracked are passive or active objects by analyzing the time it takes for the responsive signals to be received from the time the initial signal 1606 is transmitted. Since active objects will have a longer known delay amount between the transmission of signal 1606 and the receipt of the responsive signal, the module 1654 can distinguish the type of objects (e.g., passive or active) being tracked.

The position recognition module 1654 includes a user interface module that interacts with the user through the display device 1611 and input devices such as keyboard 1612 and pointing device 1614 such as arrow keys, mouse or track ball. The user interface module assists the user in programming the module 1654 to perform 3-dimensional position recognition of the objects 1608. Any of the software program modules in the program storage 1648 and data from the data storage 1650 can be transferred to the memory 1644 as needed and is executed by the CPU 1646.

One exemplary system 1640 may be 8051 microcontroller from Intel Corporation of Santa Clara, CA. However, any digital signal processor, processor or microcontroller can be used.

In one embodiment, parts of or the entire system 1640 including the input devices 1612, 1614 and display device 1611 can be incorporated into the robot system 100, 300, 600 or any suitable robotic surgical system. For example, the display 1611 can be the same as display 110, 304.

While the position recognition system 1640 has been described with reference to an infrared signal, the principles disclosed herein could be used with any optical signal as well as other signals such as ultrasound signal. Also, while only a single infrared signal has been described, the present system can transmit a set of infrared signals having either different wavelengths or waveform shapes each for reflection or transmission by a respective spherical ball to be tracked.

Although several embodiments of the invention have been disclosed in the foregoing specification, it is understood that many modifications and other embodiments of the invention will come to mind to which the invention pertains, having the benefit of the teaching presented in the foregoing description and associated drawings. It is thus understood that the invention is not limited to the specific embodiments disclosed hereinabove, and that many modifications and other embodiments are intended to be included within the scope of the appended claims.

**Claims**

1. A device (200, 326, 1672) for regenerating an infrared signal (1600) transmitted over the air for use in detecting a 3-dimensional position of an object (608), comprising:

   - an infrared signal transmitter (1602) that transmits an infrared signal (1600) over the air towards an object (608) whose position is to be determined;
   - an infrared signal detector (D1) that receives from the object (608) a responsive infrared signal in response to the transmitted infrared signal;
   - a low pass filter (1668) that receives the responsive infrared signal from the detector (D1) and outputs a filtered signal;
   - a comparator (1670) that generates a comparator output representing a logic state based on a first input receiving the responsive infrared signal from the infrared signal detector (D1) and a second input receiving the filtered signal from the low pass filter (1668),
   - an amplifier (1666) coupled between the infrared signal detector (D1) and the low pass filter (1670) and operable to generate an amplified responsive infrared signal; and **characterised by**
   - a diode (D2) connected between the amplifier (1666) and the low pass filter (1670) to reduce the voltage of the amplified responsive infrared signal by a predetermined amount.

2. The device of claim 1, wherein the infrared signal detector (D1) is configured to receive a reflection of the infrared signal (1600) transmitted by the infrared signal transmitter (1672, 1602) as the responsive infrared signal.

3. The device of claim 1, wherein the diode (D2) has a forward bias voltage of 0.4 Volt or less.

4. The device of claim 1, wherein the diode (D2) includes a Schottky diode.

5. The device of claim 1, further comprising a diode (D2) connected between the amplifier (1670) and the low pass filter, wherein the low pass filter includes a capacitor (C1) coupled between the diode (D2) and ground.

6. The device of claim 1, wherein the low pass filter includes:

   - a voltage divider (R1, R2) having first and second resistors (R1, R2) connected in series between the output of the infrared signal detector (D1) and ground, a node between the first and second resistors (R1, R2)defining an output of the voltage divider;
   - a capacitor (C1) coupled between the voltage divider output and ground.

7. The device of claim 1, further comprising a signal processor (1646) adapted to receive the comparator output (Vout) and determine a 3-dimensional position of the object (608).


**Patentansprüche**

1. Vorrichtung (200, 326, 1672) zum Regenerieren eines über die Luft übertragenen Infrarotsignals (1600) zur Verwendung beim Erfassen einer dreidimensionalen Position eines Gegenstands (608), umfassend:

   - einen Infrarotsignalsender (1602), der ein Infrarotsignal (1600) über die Luft an einen Gegenstand (608) sendet, dessen Position bestimmt werden soll;
   - einen Infrarotsignaldetektor (D1), der vom Gegenstand (608) ein Infrarotansprechsignal als Reaktion auf das gesendete Infrarotsignal empfängt;
   - einen Tiefpassfilter (1668), der das Infrarotansprechsignal vom Detektor (D1) empfängt und ein gefiltertes Signal ausgibt;
   - einen Komparator (1670), der eine Komparatorausgabe, die einen logischen Zustand darstellt, auf der Basis einer ersten Eingabe, die das Infrarotansprechsignal vom Infrarotsignaldetektor (D1) empfängt, und einer zweiten Eingabe, die das gefilterte Signal vom Tiefpassfilter (1668) empfängt, erzeugt,
   - einen Verstärker (1666), der zwischen dem Infrarotsignaldetektor (D1) und dem Tiefpassfilter (1670) gekoppelt ist, und betriebsfähig ist, um ein verstärktes Infrarotansprechsignal zu erzeugen; und **gekennzeichnet durch**

   - eine Diode (D2), die mit dem Verstärker (1666) und dem Tiefpassfilter (1670) verbunden ist, um die Spannung des verstärkten Infrarotansprechsignals um einen vorbestimmten Betrag zu verringern.

2. Vorrichtung nach Anspruch 1, wobei der Infrarotsignaldetektor (D1) eingerichtet ist, um eine Reflexion des Infrarotsignals (1600), das durch den Infrarotsignalsender (1672, 1602) übertragen wird, als Infrarotansprechsignal zu empfangen.

3. Vorrichtung nach Anspruch 1, wobei die Diode (D2) eine Durchlassvorspannung von 0,4 Volt oder weniger aufweist.

4. Vorrichtung nach Anspruch 1, wobei die Diode (D2) eine Schottky-Diode umfasst.

5. Vorrichtung nach Anspruch 1, die ferner eine Diode (D2) aufweist, die mit dem Verstärker (1670) und dem Tiefpassfilter verbunden ist, wobei der Tiefpassfilter einen Kondensator (C1) umfasst, der zwischen der Diode (D2) und Masse gekoppelt ist.

6. Vorrichtung nach Anspruch 1, wobei der Tiefpassfilter umfasst:

   - einen Spannungsteiler (R1, R2) mit ersten und zweiten Widerständen (R1, R2), die in Reihe mit der Ausgabe des Infrarotsignaldetektors (D1) und Masse verbunden sind, wobei ein Knoten zwischen dem ersten und dem zweiten Widerstand (R1, R2) ein Ausgabe des Spannungsteilers bildet;
   - einen Kondensator (C1), der zwischen der Spannungsteilerausgabe und Masse gekoppelt ist.

7. Vorrichtung nach Anspruch 1, die ferner einen Signalprozessor (1646) aufweist, der angepasst ist, um die Komparatorausgabe (Vout) zu empfangen und um eine dreidimensionale Position des Gegenstands (608) zu bestimmen.

**Revendications**

1. Dispositif (200, 326, 1672) pour la régénération d'un signal infrarouge (1600) transmis par voie aérienne, pour utilisation dans la détection d'une position tridimensionnelle d'un objet (608), comprenant :

   - un émetteur de signal infrarouge (1602) qui transmet un signal infrarouge (1600) par voie aérienne vers un objet (608) dont la position doit être déterminée ;
   - un détecteur de signal infrarouge (D1) qui reçoit en provenance de l'objet (608) un signal infrarouge de réponse en réponse au signal infrarouge transmis ;
   - un filtre passe-bas (1668) qui reçoit le signal infrarouge de réponse en provenance du détecteur (D1) et délivre en sortie un signal filtré ;
   - un comparateur (1670) qui génère une sortie de comparateur représentant un état logique sur la base d'une première entrée recevant le signal infrarouge de réponse en provenance du détecteur de signal infrarouge (D1) et d'une seconde entrée recevant le signal filtré en provenance du filtre passe-bas (1668),
   - un amplificateur (1666) couplé entre le détecteur de signal infrarouge (D1) et le filtre passe-bas (1670) et actionnable pour générer un signal infrarouge de réponse amplifié ; et **caractérisé par**
   - une diode (D2) connectée entre l'amplificateur (1666) et le filtre passe-bas (1670) pour réduire la tension du signal infrarouge de réponse amplifié, d'une quantité prédéterminée.

2. Dispositif selon la revendication 1, dans lequel le détecteur de signal infrarouge (D1) est configuré pour recevoir une réflexion du signal infrarouge (1600) transmis par l'émetteur de signal infrarouge (1672, 1602) en tant que signal infrarouge de réponse.

3. Dispositif selon la revendication 1, dans lequel la diode (D2) a une tension de polarisation directe de 0,4 V ou moins.

4. Dispositif selon la revendication 1, dans lequel la diode (D2) inclut une diode Schottky.

5. Dispositif selon la revendication 1, comprenant en outre une diode (D2) connectée entre l'amplificateur (1670) et le filtre passe-bas, dans lequel le filtre passe-bas inclut un condensateur (C1) couplé entre la diode (D2) et la terre.

6. Dispositif selon la revendication 1, dans lequel le filtre passe-bas inclut :

   - un diviseur de tension (R1, R2) ayant des première et seconde résistances (R1, R2) connectées en série entre la sortie du détecteur de signal infrarouge (D1) et la terre, un noeud entre les première et seconde résistances (R1, R2) définissant une sortie du diviseur de tension ;
   - un condensateur (C1) couplé entre la sortie de diviseur de tension et la terre.

7. Dispositif selon la revendication 1, comprenant en outre un processeur de signal (1646) adapté pour recevoir la sortie de comparateur (Vout) et déterminer une position tridimensionnelle de l'objet (608).

FIG. 1

FIG. 2

FIG. 3

FIG. 4

300

Tracking Subsystem 532

Position Sensor 540

Camera Converter 542

Computer Subsystem 504

Display 304

Speaker 536

Computer 408

Motor 510

Motor 512

Motor 514

Motor 516

Motor 518

Driver 508

Controller 538

End-Effector 310

Motion Control Subsystem 506

Stabilizer 520

Stabilizer 522

Stabilizer 524

Stabilizer 526

Platform Interface Module 406

Ring 324

Control Panel 322

Power Distribution Module 404

Battery 402

Connector Panel 320

Charging Station 534

Platform Subsystem 502

Power Supply 548

Foot Pedal 544

Tablet 546

FIG. 5

FIG. 6

FIG. 7A

FIG. 7B

FIG. 7C

FIG. 8

FIG. 9A

FIG. 9B

FIG. 9C

FIG. 10

## 1500

1502 — Import representation of targeted anatomical structure in imaging space with a detectable imaging pattern of fiducials on a registration fixture.

1504 — Detect and register the imaging pattern of fiducials of the registration fixture in the imaging space of the targeted anatomical structure.

1506 — Show representation of registration fixture overlaid on images of targeted anatomical structure.

1508 — Detect and register a navigation pattern of optical markers on the registration fixture in the navigational space of the targeted anatomical structure.

1510 — Transfer registration from imaging space to the navigational space using relative position of the imaging pattern of the registration fixture and the navigation pattern of the registration fixture.

1512 — Transfer registration from navigational space of the registration fixture to the navigational space of a dynamic reference array attached to a patient fixture instrument.

1514 — Overlay navigational space on imaging space whereby navigational markers are visible on images of targeted anatomical structure.

1516 — Track objects with optical markers wherein graphical representations of the objects are overlaid on images of the targeted anatomical structure.

**FIG. 11**

FIG. 12A

FIG. 12B

1600

1604　　　　　　　　　　　　　　　　　　1606

Power

Burst　　　　　　　　　　　　　No Burst

time

FIG. 13

1672

1664　　　　　$V_{sig}$　　　　　　　　　1670

1666

IR
Detector　　　　$V_{ref}$　　　　　$V_{out}$　to
　　　　　　　　　　　　　　　　　　　1252
Amplifiers　　D2　　R1　　C1　　R2　　Comparator

D1

1662

1608

1602　　　　　　1668

FIG. 14

FIG. 15

FIG. 16

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2015260831 A1

- US 924505 A